# EUROPÄISCHE PATENTSCHRIFT

(11) **EP 1 128 723 B1**
(45) Veröffentlichungstag und Bekanntmachung des Hinweises auf die Patenterteilung: **10.07.2002**
(21) Anmeldenummer: 99957274.6
(22) Anmeldetag: 02.11.1999
(51) Int. Cl.: A01N 25/34, A01N 25/10, A61L 29/16, A61L 29/06

(54) **WIRKSTOFFHALTIGE AROMATISCHE COPOLYESTER**
AROMATIC COPOLYESTER CONTAINING ACTIVE INGREDIENTS
COPOLYESTERS AROMATIQUES CONTENANT DES SUBSTANCES ACTIVES

(30) Priorität: 12.11.1998 DE 19852192
(43) Veröffentlichungstag der Anmeldung: 05.09.2001
(73) Patentinhaber: Bayer Aktiengesellschaft, 51368 Leverkusen (DE)
(72) Erfinder: ALBERS, Reinhard, D-51275 Leverkusen (DE); DUJARDIN, Ralf, Novi, MI 48374 (US); PUDLEINER, Heinz, D-47800 Krefeld (DE); SIMON, Joachim, D-40589 Düsseldorf (DE); FRÖDE, Rita, D-40789 Monheim (DE); HOBLER, Hartwin, D-42111 Wuppertal (DE)
(86) Internationale Anmeldenummer: EP9908354
(87) Internationale Veröffentlichungsnummer: WO0028815

(56) Entgegenhaltungen:
- WO-A-96/22114
- DE-A- 2 239 271
- US-A- 5 165 952
- US-A- 5 556 383
- PATENT ABSTRACTS OF JAPAN vol. 1997, no. 02, 28. Februar 1997 (1997-02-28) & JP 08 280790 A (OTSUKA PHARMACEUT FACTORY INC), 29. Oktober 1996 (1996-10-29) -& DATABASE WPI Week 9702 Derwent Publications Ltd., London, GB; AN 1997-015196 XP002135906 & JP 08 280790 A -& JP 08 280790 A (OTSUKA PHAMRMACEUT FACTORY INC)
- SCHIERHOLZ J M ET AL: "Controlled release of antibiotics from biomedical polyurethanes: morphological and structural features" BIOMATERIALS,GB,ELSEVIER SCIENCE PUBLISHERS BV., BARKING, Bd. 18, Nr. 12, 1. Januar 1997 (1997-01-01), Seiten 839-844, XP004064466 ISSN: 0142-9612

## Beschreibung

Die Erfindung betrifft antimikrobiell wirksame Stoffe in homogener Verteilung enthaltende aromatische Copolyester, Verfahren zu ihrer Herstellung und deren Verwendung in medizinischen Artikeln.

Vaskuläre, Katheter-assoziierte Infektionen stellen eine wichtige Ursache bei der Morbidität und Mortalität von Patienten der Intensivstation dar. Neuere Studien belegen, daß in diesem Patientenkollektiv bis 16 % der Katheterträger eine Katheterinduzierte Sepsis erleiden. Ca. 2 % dieser Patienten zeigen ernsthafte klinische Komplikationen, insbesondere einen septischen Schock oder ein akutes Organversagen. In der Zukunft ist mit einer weiter steigenden Inzidenz von Katheterinfektionen zu rechnen, da Katheter in der modernen Intensivtherapie zunehmend bei invasiven Monitoring- bzw. Behandlungsstrategien, wie z.B. der kontinuierlichen Hämofiltration Anwendung finden.

Zahlreiche Studien haben ergeben, daß koagulase-negative Staphylococcen, der transiente Keim Staphylococcus aureus und verschiedene Candida Spezies die Hauptverursacher von Katheter-assoziierten Infektionen sind. Diese ubiquitär auf der Haut vorhandenen Mikroorganismen durchdringen bei der Applikation des Katheters die physiologische Hautbarriere und gelangen so in den subkutanen Bereich und letztendlich in die Blutbahn. Die Adhäsion der Bakterien auf der Kunststoffoberfläche wird als essentieller Schritt bei der Pathogenese von Fremdkörperinfektionen betrachtet. Nach der Adhäsion der Hautkeime an der Polymeroberfläche folgt die Proliferation der Bakterien mit der Besiedlung des Polymers. Damit einher geht die Produktion eines Biofilms durch bakterielle Exkretion von extrazellulärer Glykocalix. Der Biofilm unterstützt die Adhäsion der Erreger und schützt sie vor der körpereigenen Immunabwehr. Zudem bildet der Biofilm eine für viele Antibiotika undurchdringliche Barriere. Nach verstärkter Proliferation der pathogenen Keime an der Polymeroberfläche, kann es schließlich zu einer septischen Bakteriämie kommen. Zur Therapie derartiger Infektionen ist eine Entfernung des infizierten Katheters nötig, da eine Chemotherapie mit Antibiotika unphysiologisch hohe Dosen verlangen würde.

Die Verwendung von zentralvenösen Kathetern beinhaltet daher nicht nur ein hohes Infektionsrisiko für die Patienten, sondern verursacht auch enorme therapeutische Folgekosten.

Durch prophylaktische Maßnahmen, wie z.B. hygienische Maßnahmen oder routinemäßige endoluminale Antibiotikaapplikationen kann diese Problematik nur teilweise gelöst werden. Eine rationale Strategie zur Prävention von Polymer-assoziierten Infektionen besteht in der Modifizierung der verwendeten polymeren Materialien. Ziel dieser Modifizierung muß die Hemmung der Bakterienadhäsion bzw. der Proliferation bereits adhärierter Bakterien sein, um somit kausal Fremdkörperinfektionen zu vermeiden. Dies kann z.B. durch Inkorporierung einer geeigneten antimikrobiell wirksamen Substanz in die Polymermatrix (z.B. Antibiotika) gelingen, vorausgesetzt, daß der eingearbeitete Wirkstoff auch aus der Polymermatrix herausdiffundieren kann. In diesem Fall kann die Freisetzung des Wirkstoffs auf einen längeren Zeitraum ausgedehnt werden, damit für einen entsprechend längeren Zeitraum die Bakterienadhäsion bzw. Proliferation auf dem Polymer verhindert wird.

Methoden zur Herstellung antimikrobiell ausgestatteter Polymere für medizinische Anwendungen sind bereits bekannt. In den zahlreich beschriebenen Verfahren erfolgt der Zusatz des Wirkstoffs durch folgende Techniken:
a) Adsorption auf der Polymeroberfläche (passiv oder via Surfactants)
b) Einbringen in eine Polymerbeschichtung, die auf der Oberfläche eines Formkörpers appliziert wird
c) Inkorporierung in die bulk-Phase des polymeren Trägermaterials
d) Kovalente Bindung an der Polymeroberfäche

Aus DE-A-41 43 239 geht beispielsweise ein Verfahren hervor, Wirkstoffe in die äußere Schicht medizinischer Artikel einzubringen (Imprägnierung). Dabei wird die implantierbare Vorrichtung aus polymerem Material in einem geigneten Lösungsmittel gequollen. Die Polymermatrix wird dabei so verändert, daß ein pharmazeutischer Wirkstoff bzw. eine Wirkstoffkombination in das polymere Material des Implantats eindringen kann. Nach Entfernen des Lösungsmittels wird der Wirkstoff in der Polymermatrix eingeschlossen. Nach Kontakt mit physiologischen Medium wird der in der implantierbaren Vorrichtung enthaltene Wirkstoff durch Diffusion wieder freigesetzt. Das Freisetzungsprofil kann dabei durch die Wahl des Lösungsmittels und durch Variation der experimentellen Bedingungen eingestellt werden.

Polymermaterialien für medizinische Anwendungen, die wirkstoffhaltige Beschichtungen aufweisen, werden beispielsweise in EP-A 328 421 erwähnt. Beschrieben werden Verfahren zur Hestellung der antimikrobiell wirksamen Beschichtungen sowie Methoden zur Applikation auf die Oberflächen von medizinischen Devices. Die Beschichtungen bestehen aus einer Polymermatrix, insbesondere aus Polyurethanen, Silikonen oder bioabbaubaren Polymeren, und einer antimikrobiell wirksamen Substanz, vorzugsweise einer synergistischen Kombination eines Silbersalzes mit Chlorhexidin oder einem Antibiotikum.

Ethylene-Vinyl Copolymere und Polyetherblock-Systeme mit Polyamide, Polyurethane, Polyester, welche antibakterielle Stoffe enthalten die im Polymer homogen verteilt sind, werden in JP-A-08 280790 erwähnt. Allen erwähnten Verfahren ist gemeinsam, daß das Ausrüsten des medizinischen Arbeitsmittels mit einer pharmakologisch wirksamen Substanz einen zusätzlichen Arbeitsschritt erforderlich macht, nämlich entweder eine Vorbehandlung des Polymermaterials vor der Verarbeitung oder eine Nachbehandlung der hergestellten Formkörper. Dies verursacht zusätzliche Kosten und bringt einen erhöhten Zeitaufwand bei der Produktion mit sich. Eine weitere Problematik der Verfahren besteht in der Verwendung von organischen Lösungsmittel, die meist nicht restlos aus dem Material entfernt werden können.

Aufgabe der Erfindung war es, neue Polymermaterialien bereitzustellen, die zur Herstellung von medizinischen Formkörpern für Kurzzeitimplantate, insbesondere Kathetern, geeignet sind, und für einen längeren Zeitraum (2-4 Wochen) effizient eine Oberflächenbesiedlung durch Keime verhindern.

Es wurden nun antimikrobiell wirksame Stoffe in homogener Verteilung enthaltende aromatische Copolyester gefunden, die über einen längeren Zeitraum eine die Besiedelung mit Keimen unterbindende Konzentration eines antimikrobiell wirksamen Stoffes an der Oberfläche freisetzen. Gegenstand der Erfindung sind somit aromatische Copolyester, die einen antimikrobiell wirksamen Stoff in homogener Verteilung enthalten.

Als antimikrobiell wirksame Substanzen kommen prinzipiell alle Wirkstoffe in Betracht, die ein breites Wirkungsspektrum gegen die bei Polymer-assozierten Infektionen involvierten pathogenen Mikroorganismen aufweisen, insbesondere gegen koagulase-negative Staphylokokken, Staphylokokkus aureus und Candida-Spezies. Die antimikrobiell wirksamen Substanzen können erfindungsgemäß auch als Wirkstoffkombinationen in den Formkörpern verwendet werden, sofern sich ihre Wirkungen nicht antagonisieren.

Der verwendete Wirkstoff muß eine ausreichende (chemische) Stabilität in der Polymermatrix besitzen. Zudem darf die mikrobiologische Aktivität des Wirkstoffs in der polymeren Matrix und unter den Verfahrensbedingungen der Einarbeitung nicht beeinträchtigt werden, der Wirkstoff muß also bei den für die thermoplastische Verabeitung des polymeren Materials erforderlichen Temperaturen von 150 bis 200°C stabil sein.

Die Inkorporierung der pharmazeutisch wirksamen Substanz sollte weder die Biokompatibilität der Polymeroberfäche noch andere wünschenswerte polymer-spezifische Eigenschaften des polymeren Materials (Elastizität, Reißfestigkeit etc.) beeinträchtigen.

Geeignete antibiotisch wirksame Substanzen sind beispielsweise:
- ältere Chinolone wie z.B. Nalixidinsäure, Pipemidsäure und Cinoxacin,
- neuere Chinolone wie z.B. Ciprofloxacin, Norfloxacin, Ofloxacin, Pefloxacin, Enoxacin, vorzugsweise Ciprofloxacin,
- Aminoglycoside, wie z.B. Gentamycin, Kanamycin, Amikacin, Sisomycin, Tobramycin, Netilmicin, vorzugsweise Gentamycin und Kanamycin,
- Makrolide wie z.B. Erythromycin, Clarithromycin und Azithromycin
- Polypeptide wie z.B. Bacitracin, Mupirocin, Thyrothricin (Kombination von Gramicidin und Tyrocidin,
- Lincomycine wie z.B. Lincomycin und Clindamycin
- Antimycobakterielle Mittel wie z.B. Rifampicin oder
- Fusidinsäure.

Die antimikrobiell wirksame Substanz kann auch ein Antiseptikum oder ein Desinfektionsmittel sein, solange die verwendete Substanz eine ausreichende Wirksamkeit gegen die infektionsverursachenden Spezies besitzt.

Darüber hinaus können Substanzen (pro-drugs) eingesetzt werden, die nach Einfluß mikrobieller Tätigkeit eine antimikrobiell wirksame Substanz freisetzen.

Die Wirkstoffe werden vorzugsweise in einer ihrer antimikrobiellen Aktivität entsprechenden Konzentration eingearbeitet. Besonders bevorzugt werden die Wirkstoffe in einem Konzentrationsbereich von 0,01 bis 10,0 Gew.-% verwendet.

Geeignete Copolyester (segmentierte Polyesterelastomere) sind beispielsweise aus einer Vielzahl wiederkehrender, kurzkettiger Estereinheiten und langkettiger Estereinheiten, die durch Esterbindungen vereinigt sind, aufgebaut, wobei die kurzkettigen Estereinheiten etwa 15-65 Gew.-% des Copolyesters ausmachen und die Formel (I) haben. 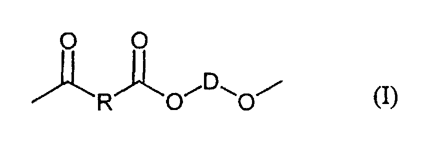 in welcher
- R: für einen zweiwertigen Rest einer Dicarbonsäure steht, der ein Molekulargewicht von unter etwa 350 hat,
- D: für einen zweiwertigen Rest eines organischen Diols steht, der ein Molekulargewicht von unter etwa 250 hat;
die langkettigen Estereinheiten machen etwa 35-85 Gew.-% des Copolyesters aus und haben die Formel II 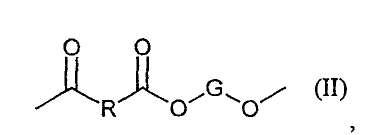 in welcher
- R: für einen zweiwertigen Rest einer Dicarbonsäure steht, der ein Molekulargewicht von unter etwa 350 hat,
- G: für einen zweiwertigen Rest eines langkettigen Glykols steht, das ein durchschnittliches Molekulargewicht von etwa 350 bis 6000 hat.

Die erfindungsgemäß verwendbaren Copolyester sind herstellbar, indem man miteinander a) eine oder mehrere Dicarbonsäuren, b) ein oder mehrere lineare, langkettige Glykole und c) ein oder mehrere niedermolekulare Diole polymerisiert.

Die Dicarbonsäuren für die Herstellung des Copolyesters sind die aromatischen Säuren mit 8-16 C-Atomen, insbesondere Phenylendicarbonsäuren, wie Phthal-, Terephthal- und Isophthalsäure.

Die niedermolekularen Diole für die Umsetzung zur Bildung der kurzkettigen Estereinheiten der Copolyester gehören den Klassen der acyclischen, alicyclischen und aromatischen Dihydroxyverbindungen an. Die bevorzugten Diole haben 2-15 C-Atome, wie Ethylen-, Propylen-, Tetramethylen-, Isobutylen-, Pentamethylen-, 2,2-Dimethyltrimethylen-, Hexamethylen- und Decamethylenglykole, Dihydroxycyclohexan, Cyclohexandimethanol, Resorcin, Hydrochinon und dergleichen. Zu den Bisphenolen für den vorliegenden Zweck gehören Bis-(p-hydroxy)-diphenyl, Bis-(phydroxyphenyl)-methan, Bis-(p-hydroxyphenyl)-ethan und Bis-(p-hydroxyphenyl)-propan.

Die langkettigen Glykole zur Herstellung der weichen Segmente der Copolyester haben vorzugsweise Molekulargewichte von etwa 600 bis 3000. Zu ihnen gehören Poly-(alkylenether)-glycole, bei denen die Alkylengruppen 2-9 Kohlenstoffatome aufweisen.

Auch Glykolester von Poly-(alkylenoxid)-dicarbonsäuren oder Polyesterglycole können als langkettiges Glykol Verwendung finden.

Zu den langkettigen Glykolen gehören auch Polyformale, die durch Umsetzung von Formaldehyd mit Glykolen erhalten werden. Auch Polythioetherglycole sind geeignet. Polybutadien- und Polyisoprenglycole, Mischpolymere derselben und gesättigte Hydrierungsprodukte dieser Materialien stellen zufriedenstellende langkettige polymere Glykole dar.

Verfahren zur Synthese derartiger Copolyester sind aus DE-OS 2 239 271, DE-OS 2 213 128, DE-OS 2 449 343 und US-A 3 023 192 bekannt.

Die beschriebenen Copolyester sind durch ein sehr gutes mechanisches Eigenschaftsprofil gekennzeichnet. Die Shore Härte kann je nach Zusammensetzung der Copolyester über einen weiten Bereich (Shore 70 A - Shore 60 D) variiert werden. Im Vergleich zu thermoplastischen Polyurethanen (TPU) liegen die Moduli der Copolyester bei gleicher Härte auf deutlich höherem Niveau.

Aufgrund dieser spezifischen Eigenschaften sind die Copolyester für die Herstellung von medizinischen Devices geeignet, insbesondere für Anwendungen bei der transluminalen Angioplastie (Ballondilatation).

Die erfindungsgemäßen wirkstoffhaltigen Copolyester können weiterhin die für Kunststoffe üblichen Additive enthalten. Übliche Additive sind beispielsweise Gleitmittel wie Fettsäureester, deren Metallseifen, Fettsäureamide und Siliconverbindungen, Antiblockmittel, Inhibitoren, Stabilisatoren gegen Hydrolyse, Licht, Hitze und Verfärbung, Flammschutzmittel, Farbstoffe, Pigmente, anorganische oder organische Füllstoffe und Verstärkungsmittel. Verstärkungsmittel sind insbesondere faserartige Verstärkungsstoffe wie anorganische Fasern, die nach dem Stand der Technik hergestellt werden und auch mit einer Schlichte beaufschlagt sein können. Nähere Angaben über die genannten Hilfs- und Zusatzstoffe sind der Fachliteratur zu entnehmen, beispielsweise R.Gächter, H.Müller (Ed.): Taschenbuch der Kunststoff-Additive, 3. Ausgabe, Hanser Verlag, München 1989, oder DE-A 29 01 774.

Systematische Untersuchungen haben ergeben, daß eine homogene Verteilung der antimikrobiell wirksamen Substanz in der Polymermatrix notwendig ist, um die Wirkstoffdiffusion als einstellbaren Freisetzungsmechanismus nutzen zu können. Die antimikrobiell wirksame Substanz und das verwendete polymere Trägermaterial sollten daher eine hohe physiko-chemische Kompatibilität besitzen. Ein Maß für die Kompatibilität von Wirkstoff und Matrix ist die im System auftretende Grenzflächenenergie. Ist diese hoch, so sind Wirkstoff und Matrix wenig kompatibel und der Wirkstoff wird rasch abgegeben. Ist die Grenzflächenenergie zu niedrig, wird der Wirkstoff von der Polymermatrix stark gebunden; eine Freisetzung effektiver Mengen an der Oberfläche unterbleibt. Bei guter physikalisch-chemischer Kompatibilität von Wirkstoff und Matrix wird ein hoher Diffusionskoeffizient des Wirkstoffs im Polymer erzielt. Die Höhe der Abgaberate der antimikrobiell wirksamen Substanz kann in diesem Fall durch Variation der eingearbeiteten Wirkstoffmenge reguliert werden, da dann die freigesetzte Menge Wirkstoff der Konzentration in der Matrix proportional ist.

Zur Herstellung der erfindungsgemäßen wirkstoffhaltigen Copolyester werden bevorzugt Kombinationen von Matrix und Wirkstoff gewählt, die eine Grenzflächenenergie von 0.5 bis 30 mN/m, besonders bevorzugt 5 bis 15 mN/m aufweisen.

Die erfindungsgemäßen wirkstoffhaltigen Copolyester zeichnen sich dadurch aus, daß sie eine molekulardisperse Verteilung der pharmakologisch wirksamen Substanz in der polymeren Matrix aufweisen. Die hohe morphologische Homogenität der extrudierten wirkstoffhaltigen Kunststoffe konnte anhand licht- und rasterelektronenmikroskopischer Aufnahmen belegt werden. Zudem konnte mit Hilfe der rastermikroskopischen Aufnahmen demonstriert werden, daß das Polymer vor und nach der Freisetzung des eingearbeiteten Wirkstoffs eine glatte Oberfläche besitzt, d.h die Biokompatibilität der Polymeroberfläche wird weder durch den Zusatz noch durch die Freisetzung des Wirkstoffs beeinträchtigt.

Auch die mechanischen Eigenschaften des Polymers werden durch den Zusatz der pharmakologisch wirksamen Substanzen in Mengen von 0,1 bis 5 Gew.-% nicht beeinträchtigt. Für bestimmte Werkstoff/Wirkstoffkombinationen wird sogar eine Verbesserung der mechanischen Eigenschaften beobachtet.

Vergleichbare wirkstoffhaltige Proben, die mittels der Gießfilmethode (solvent casting) hergestellt wurden, sind dagegen deutlich inhomogener. Rasterelektronenmikroskopische Untersuchungen belegen, daß die eingearbeiteten Wirkstoffe in der Polymermatrix und auf der Oberfäche z.T. in Form von Kristallverbänden vorliegen. Die Kristallverbände bewirken eine Verschlechterung der mechanischen Eigenschaften des Polymers. Zudem hinterlassen die herausgelösten Kristallverbände eine rauhe Oberfläche, was zu verminderter Biokompatibilität führt.

Die erfindungsgemäßen Formkörper können durch Extrudieren einer Schmelze bestehend aus dem Polymer und Wirkstoff hergestellt werden. Die Schmelze kann 0,01 bis 10 Gew-%, vorzugsweise 0,1 bis 5 Gew.-% Wirkstoff enthalten. Das Vermischen der Komponenten kann nach bekannten Techniken in jeglicher Weise erfolgen. Der Wirkstoff kann beispielsweise direkt in fester Form in die Polymerschmelze eingebracht werden. Es kann auch ein wirkstoffhaltiger Masterbatch direkt mit dem Polymer verschmolzen werden oder mit der bereits vorliegenden Polymerschmelze gemischt werden. Der Wirkstoff kann auch vor dem Schmelzen des Polymers mittels bekannter Techniken auf das Polymer aufgebracht werden (durch Tumbeln, Besprühen etc.)

Im übrigen kann die Vermischung/Homogenisierung der Komponenten nach bekannten Techniken über Kneter oder Schneckenmaschinen erfolgen, vorzugsweise in Einoder Doppelschneckenextrudern in einem Temperaturbereich zwischen 150 und 200°C.

Durch das Vermischen der Komponenten während des Extrusionsprozesses, wird eine homogene, molekulardisperse Verteilung des Wirkstoffs in der Polymermatrix erzielt, ohne daß zusätzliche Arbeitsschritte erforderlich wären.

Die Formgebung erfolgt durch die bekannten Techniken der Thermoplastverarbeitung (Spritzguß, Schlauchabzug etc). Die Formkörper sind stippenfrei, flexibel, kleben nicht und können problemlos nach den gängigen Verfahren sterilisiert werden.

### Beispiele

### Beispiel 1 (Vergleich)

### Handelsüblicher Copolyester: Hytrel® (Fa. DuPont, Wilmington, DE 19880-0709)

### Beispiel 2

10 g Wirkstoff wurden in einem Intensivmischer auf 990 g wirkstofffreies Hytrel® aufgebracht. Das wirkstoffhaltige Zylindergranulat wurde auf einem Zweiwellenextruder ZSK1 extrudiert. Es wurde eine homogene, stippenfreie Schmelze erhalten, die nach Abkühlung im Wasser/Luftbad und Stranggranulation ein einheitliches Zylindergranulat ergab.

Für mikrobiologische *in vitro* Untersuchungen sowie für die Bestimmung des Freisetzungsprofils des eingearbeiteten Wirkstoffs wurde das Granulat jeweils zu Prüfkörpern (Platten) spritzgegossen.

| **Beispiel** | **Eingearbeiteter Wirkstoff** |
|---|---|
| 2a | Kanamycin-Disulfat |
| 2b | Gentamicin-Sulfat |
| 2c | Ciprofloxacin-Betain |
| 2d | Doxycyclin-HCl |
| 2e | Clindamycin-HCl |
| 2f | Lincomycin-HCl |
| 2g | Fusidinsäure |
| 2h | Bacitracin |

### Beispiel 3

50 g Kanamycin-Disulfat wurden in einem Intensivmischer auf 950 g wirkstofffreies Hytrel® aufgebracht. Das wirkstoffhaltige Zylindergranulat wurde auf einem Zweiwellenextruder ZSK1 extrudiert. Es wurde eine homogene, stippenfreie Schmelze erhalten, die nach Abkühlung im Wasser/Luftbad und Stranggranulation ein einheitliches Zylindergranulat ergab.

### Beispiel 4

Zur Herstellung eines wirkstoffhaltigem Masterbatches wurden 380 g wirkstofffreies Zylindergranulat des Copolyesters Hytrel® in Chloroform gelöst und mit 20 g Kanamycin-Disulfat versetzt. Das Gemisch wurde erwärmt (ca. 70°C) bis eine farblose, homogene Lösung erhalten wurde. Nach Entfernen des Lösungsmittels bei 65°C/15 mbar erhielt man farblose, leicht opake Polymerplatten, welche mit Hilfe einer Häckselmaschine zerkleinert wurden.

400 g des 5 gew.-%-igen Masterbatches wurden mit 1 600 g wirkstofffreiem Zylindergranulat gemischt und auf einem Zweiwellenextruder ZSK1 extrudiert. Es wurde eine homogene, stippenfreie Schmelze erhalten, die nach Abkühlung im Wasser/Luftbad und Stranggranulation ein einheitliches Zylindergranulat ergab.

### Beispiel 5

Zur Herstellung eines wirkstoffhaltigem Masterbatches wurden 380 g wirkstofffreies Zylindergranulat des Copolyesters Hytrel® in Chloroform gelöst und mit 20 g Gentamycin-Sulfat versetzt. Das Gemisch wurde erwärmt (ca. 70°C) bis eine farblose, homogene Lösung erhalten wurde. Nach Entfernen des Lösungsmittels bei 65°C/15 mbar erhielt man farblose, leicht opake Polymerplatten, welche mit Hilfe einer Häckselmaschine zerkleinert wurden.

400 g des 5 gew.-%-igen Masterbatches wurden mit 1 600 g wirkstofffreiem Zylindergranulat gemischt und auf einem Zweiwellenextruder ZSK1 extrudiert. Es wurde eine homogene, stippenfreie Schmelze erhalten, die nach Abkühlung im Wasser/Luftbad und Stranggranulation ein einheitliches Zylindergranulat ergab.

### Beispiel 6

Zur Herstellung eines wirkstoffhaltigem Masterbatches wurden 380 g wirkstofffreies Zylindergranulat des Copolyesters Hytrel® in Chloroform gelöst und mit 20 g Bacitracin versetzt. Das Gemisch wurde erwärmt (ca. 70°C) bis eine farblose, homogene Lösung erhalten wurde. Nach Entfernen des Lösungsmittels bei 65°C/15 mbar erhielt man farblose, leicht opake Polymerplatten, welche mit Hilfe einer Häckselmaschine zerkleinert wurden.

400 g des 5 gew.-%-igen Masterbatches wurden mit 1 600 g wirkstofffreiem Zylindergranulat gemischt und auf einem Zweiwellenextruder ZSK1 extrudiert. Es wurde eine homogene, stippenfreie Schmelze erhalten, die nach Abkühlung im Wasser/Luftbad und Stranggranulation ein einheitliches Zylindergranulat ergab.

### Beispiel 7

Aus den Prüfkörperplatten der in den Beispielen 1, 2a und 2b hergestellten Materialien wurden S2-Zugstäbe gestanzt und die Festigkeitskennwerte nach DIN 53 455 ermittelt. Die Zugverformungsreste der Prüfkörper wurden in Anlehnung an DIN 53 518 bestimmt. Die Bestimmung der E-Moduli erfolgte nach DIN 53 457.

Die Ergebnisse der Untersuchungen sind in Tabelle zusammengefaßt. Sie dokumentieren, daß das thermisch-mechanische Eigenschaftsniveau von ungefülltem Hytrel® durch Zusatz von antimikrobiell wirksamen Substanzen nicht wesentlich verändert wird.

**Tabelle 1:**

| Thermisch-mechanisches Eigenschaftsniveau von ungefülltem Hytrel und von wirkstoffhaltigen Hytrel-Mustern. | | | |
|---|---|---|---|
| **Meßwerte** | **Beispiel 1** Hytrel® | **Beispiel 2a** Hytrel®+ 1 Gew.% Kanamycin | **Beispiel 2b** Hytrel®+ 1 Gew.% Gentamicin |
| Zugfestigkeit [MPa] | 13.9 | 11.5 | 12.6 |
| Bruchdehnung [%] | 934 | 681 | 755 |
| Bleibende Dehnung bei 200% Dehnung | 62 | 59 | 62 |
| E'(10°C)[MPa] | 68 | 61 | 61 |
| E'(36 °C) [MPa] | 61 | 54 | 54 |

### Beispiel 8

Die Ermittlung der Freisetzungsprofile von wirkstoffhaltigen Polymerproben erfolgte durch Elution in Millipore Wasser (0,1 % NaN₃). In einem typischen Experiment wurden dazu 5 g wirkstoffhaltige Polymerplättchen aus Hytrel® (Fläche: 1 cm²) bei 37°C mit 20 ml Millipore Wasser versetzt und mit konstanter Geschwindigkeit gerührt. In regelmäßigen Zeitabständen von 24 h wurde das Elutionsmittels für die quantitative Bestimmung des Wirkstoffgehaltes entnommen und durch frisches Millipore Wasser ersetzt. Die Quantifizierung des freigesetzten Wirkstoffs in den entsprechenden Lösungen erfolgte via HPLC-Analytik. Alle Versuchsserien wurden 2-fach durchgeführt, die quantitative Ermittlung des Wirkstoffgehaltes erfolgte jeweils als Doppelbestimmung.

Die Ergebnisse der Untersuchungen sind in Tabelle 1 zusammengefaßt. Sie dokumentieren, daß die wirkstoffhaltigen Polymeren über einen längeren Zeitraum (2 Wochen) kontinuierlich die entsprechende antimikrobiell wirksame Substanz an der Oberfläche freisetzen. Zudem konnte gezeigt werden, daß die Wirkstoffdiffusion aus dem Polymer als einstellbarer Freisetzungsmechanismus genutzt werden kann: Je höher die eingearbeitete Wirkstoffmenge, desto größer ist die aus der Polymermatrix freigesetzte Wirkstoffkonzentration im Elutionsmedium.

**Tabelle 1:**

| | | | | | | | | | | |
|---|---|---|---|---|---|---|---|---|---|---|
| Freisetzungsprofile wirkstoffhaltiger Polymerproben. Es ist jeweils die Konzentration [mg/l] des aus der Polymerprobe freigesetzten Wirkstoffs angegeben. | | | | | | | | | | |

| **Beispiel 1:** Kontrollprobe wirkstofffreies Hytrel®, **Beispiel 2c:** 1,0 Gew.-% Ciprofloxacin-Betain in Hytrel®, **Beispiel 2d:** 1,0 Gew.-% Doxycyclin-HCl in Hytrel®, **Beispiel 2e:** 1,0 Gew.-% Clindamycin-HCl in Hytrel®, **Beispiel 2f:** 1,0 Gew.-% Lincomycin-HCl in Hytrel®, **Beispiel 2g:** 1,0 Gew.-% Fusidinsäure in Hytrel® | | | | | | | | | | |
|---|---|---|---|---|---|---|---|---|---|---|
| **Zeit [h]** | **0** | **24** | **48** | **72** | **96** | **168** | **192** | **216** | **240** | **264** |
| Beispiel 1 | 0 | 0 | 0 | 0 | 0 | 0 | 0 | 0 | 0 | 0 |
| Beispiel 2c | 0 | 190 | 261 | 316 | 357 | 420 | 446 | 465 | 484 | 501 |
| Beispiel 2d | 0 | 387 | 557 | 672 | 771 | 884 | 930 | 961 | 986 | 1003 |
| Beispiel 2e | 0 | 1532 | 1899 | 2220 | 2455 | 2795 | 2897 | 2978 | 3052 | 3113 |
| Beispiel 2f | 0 | 487 | 630 | 754 | 849 | 1051 | 1105 | 1154 | 1205 | 1247 |
| Beispiel 2g | 0 | 4,3 | 6,1 | 8,3 | 10,7 | 13,4 | 16,7 | 20,0 | 23,5 | 27,5 |

### Beispiel 9

Die Prüfung der antimikrobiellen Wirksamkeit der modifizierten Polymeren erfolgte an den Bakterienstämmen *S. epidermidis* 0-47- und *S. epidermidis* 0-47+ (Fa. bioMeriuex D-72622 Nürtingen) sowie den Teststämmen *S. aureus* ATCC 25923, *S. epidermidis* ATCC 14990 und *S. saproph.* ATCC 43867.

Die Bakterienstämme wurden jeweils in einer Übernachtkultur auf Standard II-Nähragar (Fa. Merck KGaA, D-64293 Darmstadt) kultiviert und in NaCl-Lösung (0.85 %) suspendiert. Die erhaltene Bakteriensuspension mit einer Dichte von 0.5 MacFarland wurde 1:100 in NaCl-Lösung (0.85 %) verdünnt und auf Agarplatten aufgebracht (Mueller-Hinton Agar, Fa. Merck KGaA, D-64293 Darmstadt). Die 1 cm² großen Polymerproben wurden sterilisiert, unter leichtem Druck auf die Agarplatten gelegt und 20 Stunden bei 37 °C inkubiert. Nach der Inkubation wurden die Agarplatten auf Hemmhöfe kontrolliert und die Hemmhöfe ausgemessen.

Es wurden 21 Versuchsserien (3 unterschiedliche Polymermuster gegenüber 7 Teststämmen) durchgeführt. Die Ergebnisse des Agardiffusionstests sind in Tabelle 2 zusammengefaßt. Sie zeigen, daß um die wirkstoffhaltigen Polymermuster im Vergleich zu der wirkstofffreien Probe eine Hemmzone ausgebildet wurde, in der kein Bakterienwachstum stattfindet, d.h. die wirkstoffhaltigen Polymerproben weisen eine substantielle antimikrobielle Wirkung gegenüber den verwendeten Teststämmen auf.

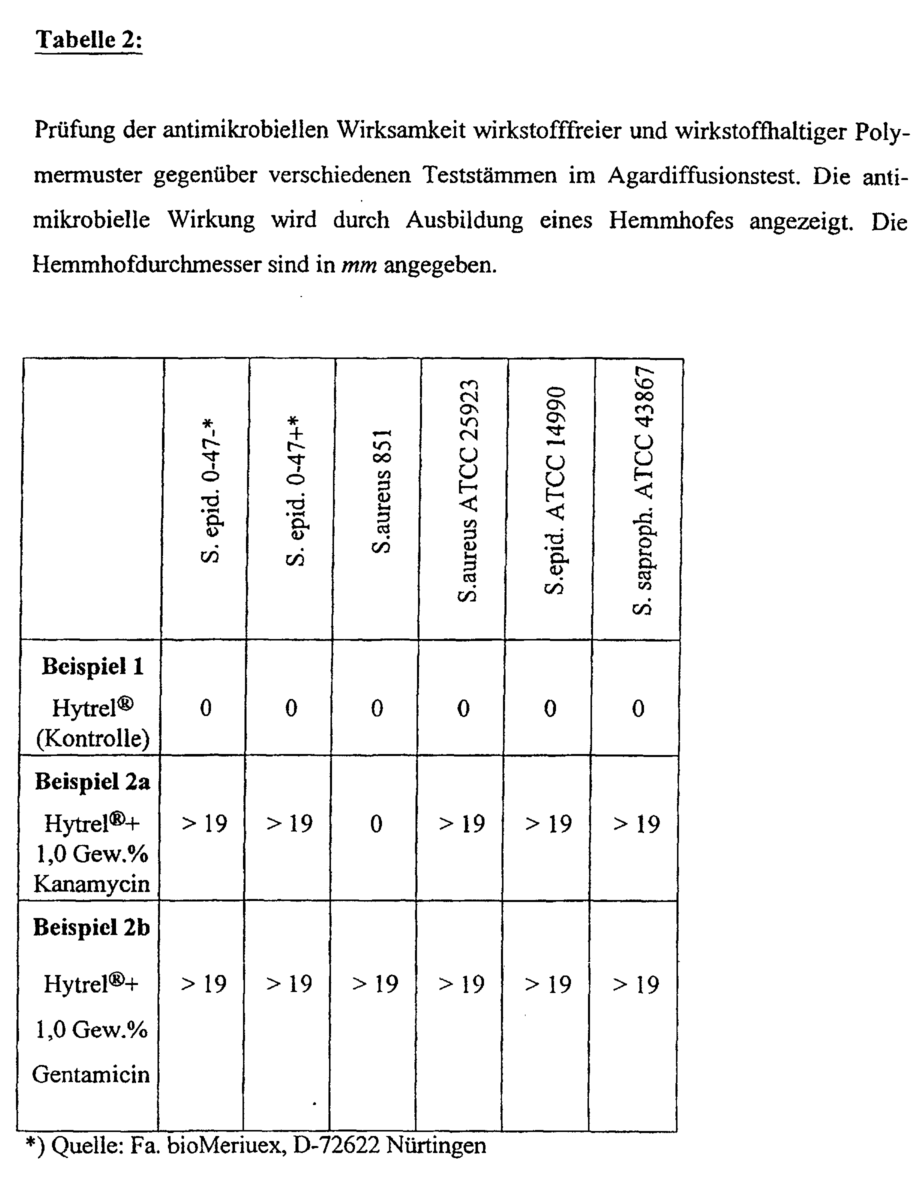

## Patentansprüche

1. Antimikrobiell wirksame Stoffe in homogener Verteilung enthaltende aromatische Copolyester.

2. Verfahren zur Herstellung der antimikrobiell wirksame Stoffe in homogener Verteilung enthaltenden aromatischen Copolyester gemäß Anspruch 1 durch homogenes Vermischen eines antimikrobiell wirksamen Stoffs und eines aromatischen Copolyesters.

3. Formkörper aus antimikrobiell wirksame Stoffe in homogener Verteilung enthaltenden aromatischen Copolyestern.

4. Verwendung der antimikrobiell wirksame Stoffe in homogener Verteilung enthaltenden aromatischen Copolyester gemäß Anspruch 1 in medizinischen Artikeln.

## Claims

1. Aromatic copolyesters containing homogeneously distributed antimicrobially active substances.

2. A process for the production of the aromatic copolyesters containing homogeneously distributed antimicrobially active substances according to claim 1 by homogeneously mixing an antimicrobially active substance and an aromatic copolyester.

3. Mouldings made from aromatic copolyesters containing homogeneously distributed antimicrobially active substances.

4. Use of the aromatic copolyesters containing homogeneously distributed antimicrobially active substances according to claim 1 in medical articles.

## Revendications

1. Copolyesters aromatiques contenant en répartition homogène des substances à activité antimicrobienne.

2. Procédé pour la préparation des copolyesters aromatiques contenant en répartition homogène des substances à activité antimicrobienne selon la revendication 1, par mélange homogène d'une substance à activité antimicrobienne et d'un copolyester aromatique.

3. Objets moulés consistant en copolyesters aromatiques contenant en répartition homogène des substances à activité antimicrobienne.

4. Utilisation des copolyesters aromatiques contenant en répartition homogène des substances à activité antimicrobienne selon la revendication 1 dans des articles médicinaux.
